(19) Europäisches Patentamt
European Patent Office
Office européen des brevets

(11) Veröffentlichungsnummer: **0 091 054**
**B1**

(12) # EUROPÄISCHE PATENTSCHRIFT

(45) Veröffentlichungstag der Patentschrift: **02.10.85**

(21) Anmeldenummer: **83103050.7**

(22) Anmeldetag: **28.03.83**

(51) Int. Cl.⁴: **C 07 C 119/048,** C 07 C 118/02, C 08 G 18/76, A 01 N 47/40

(54) **1-Alkyl-2-isocyanatomethyl-isocyanato-benzole und/oder 1-Alkyl-4-isocyanatomethyl-isocyanato-benzole, Verfahren zu deren Herstellung und Verwendung.**

(30) Priorität: **07.04.82 DE 3212927**

(43) Veröffentlichungstag der Anmeldung: **12.10.83 Patentblatt 83/41**

(45) Bekanntmachung des Hinweises auf die Patenterteilung: **02.10.85 Patentblatt 85/40**

(84) Benannte Vertragsstaaten: **AT BE CH DE FR GB IT LI NL**

(56) Entgegenhaltungen:
**GB - A - 907 559**
**GB - A - 1 545 988**

(73) Patentinhaber: **BASF Aktiengesellschaft, Carl-Bosch-Strasse 38, D-6700 Ludwigshafen (DE)**

(72) Erfinder: **Hahn, Erwin, Dr., Am Buechsenackerhang 31, D-6900 Heidelberg (DE)**
Erfinder: **Neumann, Peter, Dr., Franz-Schubert-Strasse 1, D-6908 Wiesloch (DE)**

**EP 0 091 054 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aromatische Diisocyanate, wie z. B. 2,4- und 2,6-Toluylen-diisocyanat, 1,5-Naphthylen-diisocyanat, 4,4'-Diphenylmethan-diisocyanat und Mischungen aus 4,4'-, 2,4'- und 2,2'-Diphenylmethan-diisocyanaten und Polyphenyl-polymethylen-polyisocyanaten, aliphatische Diisocyanate, wie z. B. Hexamethylen-diisocyanat-1,6 und cycloaliphatische Diisocyanate, wie z. B. 3-Isocyanatomethyl-3,3,5-trimethyl-cyclohexylisocyanat, sind bekannte Handelsprodukte, die vorzugsweise zur Herstellung von Polyurethankunststoffen Verwendung finden. Die Polyisocyanate werden üblicherweise aus den entsprechenden Aminoverbindungen durch Phosgenierung und anschließende thermische Spaltung der intermediär gebildeten Carbaminsäurechloride hergestellt. Zahlreiche organische Mono- und Polyisocyanate werden beispielsweise beschrieben in Annalen der Chemie 562 (1949), Seiten 75ff.

Para- und meta-Isocyanato-benzylisocyanate, ein Verfahren zu deren Herstellung und ihre Verwendung zur Herstellung von Polyurethanen wird in der GB-A-1 545 988 beschrieben. Die genannten Diisocyanate besitzen Isocyanatgruppen unterschiedlicher Reaktivität und sind insgesamt wesentlich reaktiver als beispielsweise Toluylen-diisocyanat. Nachteilig ist jedoch, daß sich ihr Dampfdruck ebenfalls in ungefähr der Größenordnung von Toluylen-diisocyanat bewegt, so daß bei der Verarbeitung aus physiologischen Gründen entsprechende Vorsichtsmaßnahmen strikt eingehalten werden müssen.

Aufgabe der vorliegenden Erfindung war es, aromatische Diisocyanate zu entwickeln, die Isocyanatgruppen mit einer unterschiedlichen Reaktivität gebunden enthalten.

Gegenstand der Erfindung sind aromatische Diisocyanate der Formeln

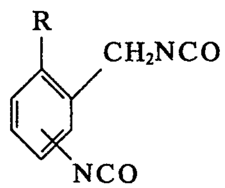

oder

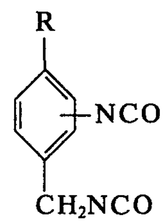

(I) (II)

in denen R eine Alkylgruppe mit 1 bis 12 Kohlenstoffatomen bedeutet.

Die erfindungsgemäßen Diisocyanate erfüllen das genannte Erfordernis.

Bevorzugt sind aromatische Diisocyanate der Formeln

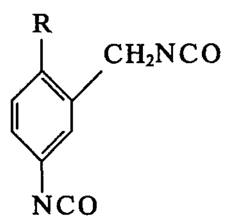

oder

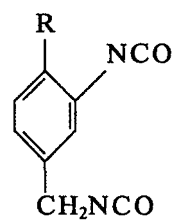

(III) (IV)

bei denen die Isocyanato-methylgruppe in 2- oder 4-Stellung und die Isocyanatgruppe in 4- oder 2-Stellung gebunden sind.

Der Alkylrest R, der aus 1 bis 12 Kohlenstoffatomen, vorzugsweise 1 bis 4 Kohlenstoffatomen besteht, kann linear oder verzweigt sein. Genannt seien beispielsweise Alkylreste, wie der n-Pentyl-, n-Hexyl-, n-Heptyl-, n-Octyl-, 2-Ethyl-n-octyl-, n-Nonyl-, n-Decyl-, n-Undecyl-, n-Dodecyl-rest, vorzugsweise der Ethyl-, n- und iso-Propyl- und n- und sek.-Butylrest und insbesondere der Methylrest.

Als erfindungsgemäße aromatische Diisocyanate kommen im einzelnen beispielsweise in Betracht:

1-Methyl-2-isocyanatomethyl-3-isocyanat-, 1-Methyl-2-isocyanatomethyl-5-isocyanato-,
1-Methyl-2-isocyanatomethyl-6-isocyanato-, 1-Methyl-4-isocyanatomethyl-3-isocyanato-benzol,
1-Ethyl-2-isocyanatomethyl-3-isocyanato-, 1-Ethyl-2-isocyanatomethyl-5-isocyanato-,
1-Ethyl-2-isocyanatomethyl-6-isocyanato-, 1-Ethyl-4-isocyanatomethyl-3-isocyanato-benzol,
1-n-Propyl-2-isocyanatomethyl-3-isocyanato-, 1-n-Propyl-2-isocyanatomethyl-5-isocyanato-,
1-n-Propyl-2-isocyanatomethyl-6-isocyanato-,
1-n-Propyl-4-isocyanatomethyl-3-isocyanato-benzol,
1-iso-Propyl-2-isocyanatomethyl-3-isocyanato-, 1-iso-Propyl-2-isocyanatomethyl-4-isocyanato-,
1-iso-Propyl-2-isocyanatomethyl-5-isocyanato-, 1-iso-Propyl-2-isocyanatomethyl-6-isocyanato-,
1-iso-Propyl-4-isocyanatomethyl-2-isocyanato-,
1-iso-Propyl-4-isocyanatomethyl-3-isocyanato-benzol,

1-n-Butyl-2-isocyanatomethyl-3-isocyanato-, 1-n-Butyl-2-isocyanatomethyl-4-isocyanato-,
1-n-Butyl-2-isocyanatomethyl-5-isocyanato-, 1-n-Butyl-2-isocyanatomethyl-6-isocyanato-,
1-n-Butyl-4-isocyanatomethyl-2-isocyanato-, 1-n-Butyl-4-isocyanatomethyl-3-isocyanato-benzol,
1-n-Pentyl-2-isocyanatomethyl-4-isocyanato-, 1-n-Pentyl-2-isocyanatomethyl-6-isocyanato-,
1-n-Pentyl-4-isocyanatomethyl-2-isocyanato-benzol,
1-n-Hexyl-2-isocyanatomethyl-4-isocyanato-, 1-n-Hexyl-2-isocyanatomethyl-6-isocyanato-,
1-n-Hexyl-4-isocyanatomethyl-2-isocyanato-benzol,
1-n-Heptyl-2-isocyanatomethyl-4-isocyanato-, 1-n-Heptyl-2-isocyanatomethyl-6-isocyanato-,
1-n-Heptyl-4-isocyanatomethyl-2-isocyanato-benzol,
1-n-Octyl-2-isocyanatomethyl-4-isocyanato-, 1-n-Octyl-2-isocyanatomethyl-6-isocyanato-,
1-n-Octyl-4-isocyanatomethyl-2-isocyanato-benzol, 1-n-Nonyl-2-isocyanatomethyl-4-isocyanato-,
1-n-Nonyl-2-isocyanatomethyl-6-isocyanato-,
1-n-Nonyl-4-isocyanatomethyl-2-isocyanato-benzol,
1-n-Decyl-2-isocyanatomethyl-4-isocyanato-, 1-n-Decyl-2-isocyanatomethyl-6-isocyanato-,
1-n-Decyl-4-isocyanatomethyl-2-isocyanato-benzol,
1-n-Undecyl-2-isocyanatomethyl-4-isocyanato-, 1-n-Undecyl-2-isocyanatomethyl-6-isocyanato-,
1-n-Undecyl-4-isocyanatomethyl-2-isocyanato-benzol,
1-n-Dodecyl-2-isocyanatomethyl-4-isocyanato-, 1-n-Dodecyl-2-isocyanatomethyl-6-isocyanato-
und 1-n-Dodecyl-4-isocyanatomethyl-2-isocyanato-benzol.

Als bevorzugt genannt seien:

1-Ethyl-2-isocyanatomethyl-4-isocyanato-,
1-Ethyl-4-isocyanatomethyl-2-isocyanato-benzol,
1-n-Propyl-2-isocyanatomethyl-4-isocyanato- und
1-n-Propyl-4-isocyanatomethyl-2-isocyanato-benzol sowie insbesondere
1-Methyl-2-isocyanatomethyl-4-isocyanato-,
1-Methyl-2-isocyanatomethyl-6-isocyanato-benzol sowie deren Isomerengemische und
1-Methyl-4-isocyanatomethyl-2-isocyanato-benzol.

Die erfindungsgemäßen aromatischen Diisocyanate können in Form von Isomerengemischen, Gemische gleicher Isomeren aber verschiedener Alkylreste oder als Mischungen von beiden Gemischen vorliegen.

Die Herstellung der neuen Diisocyanate kann beispielsweise nach folgendem Verfahrensschema erfolgen:

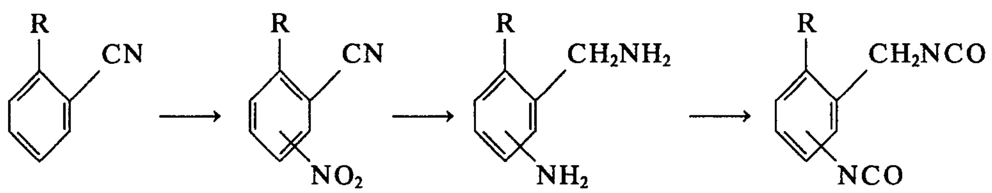

(VII) (VIII) (V) (I)

Die 1-Alkyl-2-cyano-nitrobenzole (VIII) bzw. 1-Alkyl-4-cyano-nitrobenzole können nach an sich bekannten Verfahren durch Nitrierung der entsprechenden 1-Alkyl-2-cyanobenzole (VII) oder 1-Alkyl-4-cyano-benzole hergestellt werden, beispielsweise analog den in Berichte der Deutschen Chemischen Gesellschaft 31, Seiten 2880ff (1898) oder in J. Amer. Chem. Soc. 99, 6721 (1977) beschriebenen Methoden oder geeigneten Varianten davon.

Eine andere, beispielhaft genannte Methode zur Herstellung von Alkyl-cyano-nitro-benzolen, besteht in dem bekannten Austausch der Aminogruppe von Alkyl-amino-nitrobenzolen gegen eine Nitrilgruppe, entsprechend J. Org. Chemistry 44, 4003 (1979).

Die bei der Nitrierung anfallenden 1-Alkyl-cyano-nitro-benzole oder deren Isomerengemische können direkt, d. h. ohne weitere Reinigung, z. B. analog dem in Farmaco (Pavia), Ediz. Sci. 25, 163 (1970) (C.A. 72, 121 101 d, 1970) für 2-Cyano-nitro-toluol beschriebenen Verfahren, zu 1-Alkyl-aminomethyl-amino-benzolen oder deren Isomerengemische reduziert werden. Die Reduktion der Nitro- und der Cyanogruppe kann hierbei in einem oder in zwei aufeinanderfolgenden Reaktionsschritten durchgeführt werden. Der Zusatz von Ammoniak ist nicht immer erforderlich.

Die erhaltenen 1-Alkyl-aminomethyl-amino-benzole können direkt oder als Salze, vorzugsweise Hydrochloride, in Lösungsmitteln phosgeniert werden. Geeignete Lösungsmittel sind beispielsweise Toluol, Xylol, Chlorbenzol oder Dichlorbenzol. Eine Lösung der 1-Alkyl-aminomethyl-amino-benzole oder eine Suspension der entsprechenden Salze wird danach bei Temperaturen von ungefähr 0°C bis 100°C, vorzugsweise 10 bis 50°C mit 1 bis 3 Mol, vorzugsweise 1,1 bis 1,5 Mol Phosgen pro $NH_2$- oder $NH_2 \cdot HCl$-Gruppen zur Reaktion gebracht und das intermediär gebildete Carbaminsäurechlorid bei

Temperaturen von 80 bis 180° C, vorzugsweise 120 bis 160° C in das 1-Alkyl-isocyanatomethyl-isocyanato-benzol gespalten. Das gasförmige oder flüssige Phosgen wird hierbei der Reaktionsmischung mit einer solchen Geschwindigkeit zugeführt, daß die austretenden Gase überwiegend aus Chlorwasserstoff bestehen.

Nach beendeter Phosgenierung und Spaltung wird das Lösungsmittel, vorzugsweise unter vermindertem Druck, beispielsweise von 100 bis 10 mbar abdestilliert. Gegebenenfalls kann es auch vorteilhaft sein, den Chlorwasserstoff und evtl. vorliegendes überschüssiges Phosgen mit Hilfe von Stickstoff oder einem anderen Inertgas aus der Diisocyanatlösung auszutreiben, bevor das Lösungsmittel abdestilliert wird.

Die erhaltenen rohen 1-Alkyl-2-bzw. -4-isocyanatomethyl-isocyanato-benzole oder Isomerengemische können durch Destillation unter vermindertem Druck getrennt und gereinigt werden.

Die erfindungsgemäßen aromatischen Diisocyanate können ferner durch thermische Spaltung der entsprechenden Diurethane in der Gas- oder Flüssigphase, gegebenenfalls in Gegenwart von Katalysatoren hergestellt werden, wobei die Diurethane zweckmäßigerweise nach dem Verfahren der EP-OS 18 583 (US 4 278 805) durch Umsetzung von Carbamidsäureester mit den 1-Alkyl-aminomethyl-amino-benzolen in Gegenwart von Alkoholen und gegebenenfalls Harnstoff erhalten werden.

Die neuen 1-Alkyl-2- bzw. -4-isocyanatomethyl-isocyanato-benzole sind wertvolle Ausgangsmaterialien für Pflanzenschutzmittel und Kunststoffe. Die Produkte eignen sich insbesondere zur Herstellung von Polyurethan-schaumstoffen, -klebstoffen, -lacken, -beschichtungsmitteln und -dichtungsmitteln.

Die folgenden Beispiele sollen die Erfindung näher erläutern.

## Beispiel 1

### a) Herstellung eines Isomerengemisches aus 1-Methyl-2-cyano-4-nitrobenzol und 1-Methyl-2-cyano-nitrobenzol

In einem Reaktionsgefäß, ausgestattet mit Tropftrichter, Rührer und Thermometer, wurden 1200 Gewichtsteile konz. Schwefelsäure auf −5° C abgekühlt. Unter gutem Rühren ließ man hierzu innerhalb von einer Stunde 200 Gewichtsteile 2-Cyano-toluol und danach zwischen −5° C und +5° C 151 Gewichtsteile konzentrierte Salpetersäure hinzutropfen. Zur Vervollständigung der Nitrierung rührte man noch 1,5 Stunden bei 0° C und goß das Reaktionsgemisch nun auf 2000 Gewichtsteile Eis. Man saugte den Niederschlag ab, wusch das Filtergut gründlich mit Wasser und trocknete auf Ton. Man erhielt 282 Gewichtsteile eines rohen Isomerengemisches aus 1-Methyl-2-cyano-4- und -6-nitrobenzol im Gewichtsverhältnis von ungefähr 87 : 13 mit einem Schmelzpunkt von 91 bis 92° C.

### b) Herstellung einer Isomerenmischung aus 1-Methyl-2-aminomethyl-4- und -6-amino-benzol

100 Gewichtsteile des gemäß (a) erhaltenen 1-Methyl-2-cyano-4- und -6-nitrobenzol-Isomerengemisches wurden in 800 Gewichtsteilen Ethanol gelöst und nach Zugabe von 25 Gewichtsteilen Raney-Nikkel bei 50 bar und 80° C hydriert. Nach dem Abkühlen auf Raumtemperatur wurde der Katalysator abfiltriert und das Filtrat unter vermindertem Druck eingeengt. Man erhielt 80 Gewichtsteile rohen Gemisches aus 1-Methyl-2-aminomethyl-4-und -6-amino-benzol in Form eines Öls, das rasch erstarrte.

$C_8H_{12}N_2$ (Molekulargewicht 136, massenspektrometrisch)

### c) Herstellung einer Isomerenmischung aus 1-Methyl-2-isocyanatomethyl-4- und -6-isocyanato-benzol

Zu einer auf 0° C abgekühlten Mischung aus 520 Gewichtsteilen o-Dichlorbenzol und 160 Gewichtsteilen Phosgen ließ man unter Rühren eine auf 50 bis 60° C erwärmte Lösung von 27,2 Gewichtsteilen der gemäß (b) erhaltenen Isomerenmischung aus 1-Methyl-2-aminomethyl-4- und -6-amino-benzol in 200 Gewichtsteilen o-Dichlorbenzol hinzutropfen. Nach beendeter Zugabe wurde langsam auf 130° C erwärmt und bei dieser Temperatur 1,5 Stunden lang Phosgen durch das Reaktionsgemisch geleitet. Danach ließ man das Reaktionsgemisch auf Raumtemperatur abkühlen und trennte das überschüssige Phosgen durch Einleiten von Stickstoff ab. Danach wurde zunächst das Lösungsmittel bei 10 bis 20 mbar abdestilliert und darauf der Rückstand im Hochvakuum (0,05 mbar) destilliert. Man erhielt 19,2 Gewichtsteile einer Mischung aus 1-Methyl-2-isocyanatomethyl-4- und -6-isocyanato-benzol im Gewichtsverhältnis von 87 : 13 mit einem Siedepunkt von 100 bis 105° C (0,05 mbar).

Analyse: $C_{10}H_8N_2O_2$ (Molekulargewicht: 188, massenspektrometrisch)

| | | | | |
|---|---|---|---|---|
| ber.: | C 63,83 | H 4,28 | O 14,89 | N 17,00; |
| gef.: | C 63,7 | H 4,4 | O 14,8 | N 16,6. |

Beispiel 2

a) Herstellung von 1-Methyl-4-cyano-2-nitrobenzol

In einem Reaktionsgefäß, ausgestattet mit Tropftrichter, Rührer und Thermometer, wurden 240 Gewichtsteile konz. Schwefelsäure auf —5° C gekühlt. Unter gutem Rühren trug man zunächst portionsweise 40 Gewichtsteile 4-Cyanotoluol ein und ließ danach zwischen —5° C und +5° C 25 Volumenteile konzentrierter Salpetersäure hinzutropfen. Zur Vervollständigung der Nitrierung rührte man noch 2 Stunden bei 0° C und goß das Reaktionsgemisch nun auf 400 Gewichtsteile Eis. Man saugte den Niederschlag ab, wusch das Filtergut gründlich mit Wasser und trocknete an der Luft. Man erhielt 50,9 Gewichtsteile 1-Methyl-4-cyano-2-nitrobenzol mit einem Schmelzpunkt von 105—106° C.

b) Herstellung von 1-Methyl-4-aminomethyl-2-aminobenzol

50 Gewichtsteile des gemäß a) erhaltenen 1-Methyl-4-cyano-2-nitro-benzols wurden in 500 Gewichtsteilen Ethanol gelöst und nach Zugabe von 15 Gewichtsteilen Raney-Nickel bei 10 bar und 60° C hydriert bis die Wasserstoffaufnahme beendet war. Nach Zugabe von 5 Volumenteilen 3%iger Ammoniaklösung wurde erneut bei 10 bar und 60° C hydriert. Nach dem Abkühlen auf Raumtemperatur wurde der Katalysator abfiltriert und das Filtrat unter vermindertem Druck eingeengt. Man erhielt 37,9 Gewichtsteile rohes 1-Methyl-4-aminomethyl-2-amino-benzol in Form eines Öls, das beim Stehen kristallisierte.

c) Herstellung von 1-Methyl-4-isocyanatomethyl-2-isocyanatobenzol

Zu einer auf 0° C abgekühlten Mischung aus 260 Gewichtsteilen o-Dichlorbenzol und 80 Gewichtsteilen Phosgen ließ man unter Rühren eine Lösung aus 13,6 Gewichtsteilen des gemäß b) erhaltenen 1-Methyl-4-aminomethyl-2-aminobenzols in 100 Gewichtsteilen o-Dichlorbenzol tropfen. Nach beendeter Zugabe wurde langsam auf 160° C erwärmt und bei dieser Temperatur 2 Stunden lang Phosgen durch das Reaktionsgemisch geleitet. Danach ließ man das Reaktionsgemisch auf Raumtemperatur abkühlen und trennte das überschüssige Phosgen durch Einleiten von Stickstoff ab. Danach wurde zunächst das Lösungsmittel bei 10 bis 20 mbar abdestilliert und darauf der Rückstand im Hochvakuum (0,05 mbar) destilliert. Man erhielt 9,6 Gewichtsteile 1-Methyl-4-isocyanatomethyl-2-isocyanatobenzol mit einem Siedepunkt von 103—105° C (0,05 mbar).

**Patentansprüche für die Vertragsstaaten: BE, CH, DE, FR, GB, IT, LI, NL**

1. Aromatische Diisocyanate der Formeln

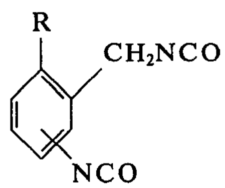

oder

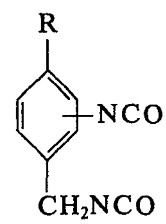

(I) (II)

in denen R eine Alkylgruppe mit 1 bis 12 Kohlenstoffatomen bedeutet.

2. Aromatische Diisocyanate der Formeln

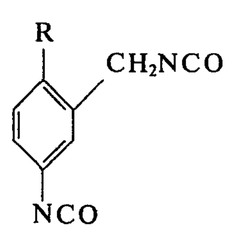

oder

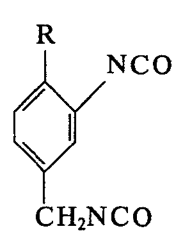

(III) (IV)

in denen R eine Alkylgruppe mit 1 bis 12 Kohlenstoffatomen bedeutet.

3. Mischungen aus aromatischen Diisocyanaten der Formeln (I) und/oder (II).

4. Mischungen aus aromatischen Diisocyanaten der Formeln (III) und/oder (IV).

5. Aromatische Diisocyanate der Formeln (III) oder (IV) in denen R eine Methyl-, Ethyl-, n-Propyl-, iso-Propyl- oder n-Butylgruppe ist.

6. Mischung aus 1-Methyl-2-isocyanatomethyl-4-isocyanato-benzol und 1-Methyl-2-isocyanatomethyl-6-isocyanato-benzol.

7. Methyl-2-isocyanatomethyl-4-isocyanato-benzol.

8. 1-Methyl-2-isocyanatomethyl-6-isocyanato-benzol.

9. 1-Methyl-4-isocyanatomethyl-2-isocyanato-benzol.

10. Verfahren zur Herstellung von aromatischen Diisocyanaten der Formeln (I) oder (II), dadurch gekennzeichnet, daß man aromatische Diamine der Formeln

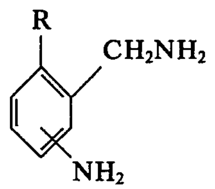

oder

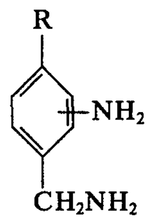

(V) (VI)

in denen R eine Alkylgruppe mit 1 bis 12 Kohlenstoffatomen bedeutet, phosgeniert und die hierbei entstehenden Carbaminsäurechloride thermisch spaltet.

11. Verwendung von aromatischen Diisocyanaten der Formel (I) und/oder (II) zur Herstellung von Polyurethan-Kunststoffen oder Pflanzenschutzmitteln.

**Patentansprüche für den Vertragsstaat: AT**

1. Verfahren zur Herstellung von aromatischen Diisocyanaten der Formeln

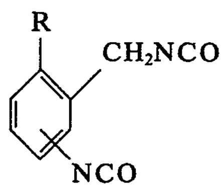

und/oder

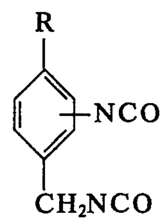

(I) (II)

dadurch gekennzeichnet, daß man aromatische Diamine der Formeln

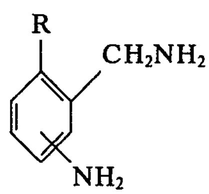

und/oder

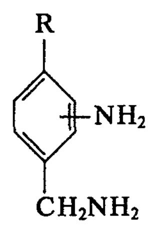

(V) (VI)

in denen R eine Alkylgruppe mit 1 bis 12 Kohlenstoffatomen bedeutet, phosgeniert und die hierbei entstehenden Carbaminsäurechloride thermisch spaltet.

2. Verfahren nach Anspruch 1, dadurch gekennzeichnet, daß R eine Methyl-, Ethyl-, n-Propyl-, iso-Propyl- oder n-Butylgruppe ist.

3. Verfahren zur Herstellung von aromatischen Diisocyanaten der Formeln (I) und/oder (II) nach Anspruch 1, dadurch gekennzeichnet, daß man als aromatische Diamine Verbindungen der Formeln

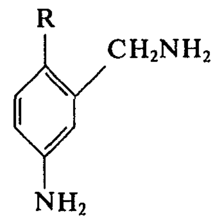

und/oder

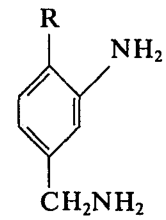

verwendet, in denen R eine Alkylgruppe mit 1 bis 12 Kohlenstoffatomen bedeutet.

4. Verfahren zur Herstellung von aromatischen Diisocyanaten der Formel (I) nach Anspruch 1, dadurch gekennzeichnet, daß man als aromatische Diamine 1-Methyl-2-aminomethyl-4-amino-benzol und/oder 1-Methyl-2-aminomethyl-6-amino-benzol verwendet.

5. Verfahren zur Herstellung von aromatischen Diisocyanaten der Formel (II) nach Anspruch 1, dadurch gekennzeichnet, daß man als aromatisches Diamin 1-Methyl-4-aminomethyl-2-amino-benzol verwendet.

**Claims for the Contracting states: BE, CH, DE, FR, GB, IT, LI, NL**

1. An aromatic diisocyanate of the formula

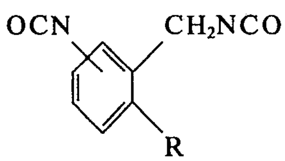

(I)

or

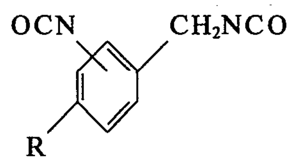

(II)

in which formulae R is alkyl of 1 to 12 carbon atoms.

2. An aromatic diisocyanate of the formula

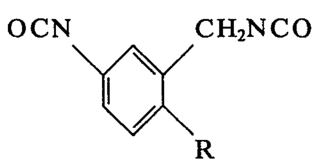

(III)

or

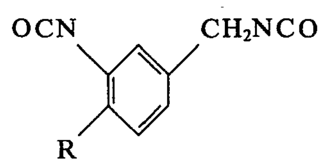

(IV)

in which formulae R is alkyl of 1 to 12 carbon atoms.

3. A mixture of aromatic diisocyanates of the formulae (I) and/or (II).

4. A mixture of aromatic diisocyanates of the formulae (III) and/or (IV).

5. An aromatic diisocyanate of the formula (III) or (IV), in which formulae R is methyl, ethyl, n-propyl, iso-propyl or n-butyl.

6. A mixture of 1-methyl-2-isocyanatomethyl-4-isocyanato-benzene and 1-methyl-2-isocyanatomethyl-6-isocyanatobenzene.

7. 1-Methyl-2-isocyanatomethyl-4-isocyanatobenzene.

8. 1-Methyl-2-isocyanatomethyl-6-isocyanatobenzene.

9. 1-Methyl-4-isocyanatomethyl-2-isocyanatobenzene.

10. A process for the preparation of an aromatic diisocyanate of the formula (I) or (II), wherein an aromatic diamine of the formula

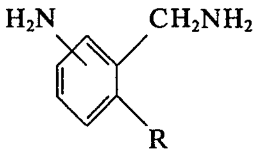

(V)

or

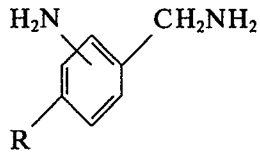

(VI)

in which formulae R is alkyl of 1 to 12 carbon atoms, is phosgenated, and the resulting carbamyl chloride is thermally cleaved.

11. The use of aromatic diisocyanates of the formulae (I) and/or (II) for the production of polyurethane plastics or plant protection products.

**Claims for the Contracting state: AT**

1. A process for the preparation of aromatic diisocyanates of the formulae

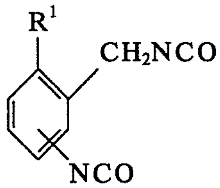

and/or

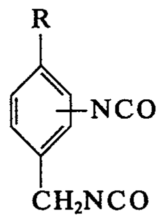

(I) (II)

wherein aromatic diamines of the formulae

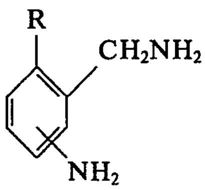

and/or

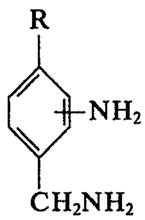

(V) (VI)

in which formulae R is alkyl of 1 to 12 carbon atoms, are phosgenated, and the resulting carbamyl chlorides are thermally cleaved.

2. A process as claimed in claim 1, wherein R is methyl, ethyl, n-propyl, isopropyl or n-butyl.

3. A process for the preparation of aromatic diisocyanates of the formulae (I) and/or (II) as claimed in claim 1, wherein compounds of the formulae

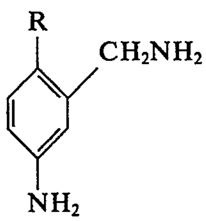

and/or

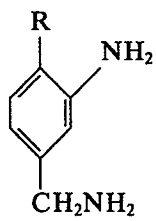

in which formulae R is alkyl of 1 to 12 carbon atoms, are used as aromatic diamines.

4. A process for the preparation of aromatic diisocanates of the formula (I) as claimed in claim 1, wherein 1-methyl-2-aminomethyl-4-aminobenzene and/or 1-methyl-2-aminomethyl-6-aminobenzene are used as aromatic diamines.

5. A process for the preparation of aromatic diisocyanates of the formula (II) as claimed in claim 1, wherein 1-methyl-4-aminomethyl-2-aminobenze is used as aromatic diamine.

**Revendications pour les Etats contractants: BE, CH, DE, FR, GB, IT, LI, NL**

1. Diisocyanates aromatiques de formules

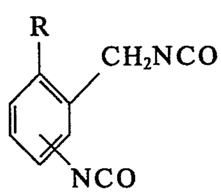

ou

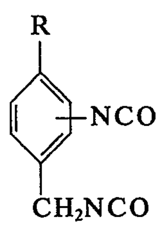

(I) (II)

dans lesquelles R représente un groupe alkyle ayant 1 à 12 atomes de carbone.

2. Diisocyanates aromatiques de formules

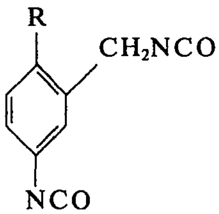

ou

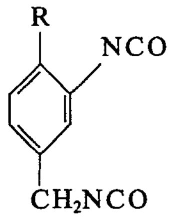

(III) (IV)

dans lequelles R représente un groupe alkyle ayant 1 à 12 atomes de carbone.

3. Mélanges de diisocyanates aromatiques de formules (I) et/ou (II).

4. Mélanges de diisocyanates aromatiques de formules (III) et/ou (IV).

5. Diisocyanates aromatiques de formules (III) ou (IV), dans lesquelles R représente un groupe méthyle, éthyle, n-propyle, iso-propyle ou n-butyle.

6. Mélange de 1-méthyl-2-isocyanatométhyl-4-isocyanato-benzène et 1-méthyl-2-isocyanatométhyl-6-isocyanato-benzène.

7. 1-méthyl-2-isocyanatométhyl-4-isocyanato-benzène.

,8. 1-méthyl-2-isocyanatométhyl-6-isocyanato-benzène.

9. 1-méthyl-4-isocyanatométhyl-2-isocyanato-benzène.

10. Procédé de préparation de diisocyanates aromatiques de formules (I) ou (II), caractérisé par le fait que l'on soumet à l'action du phosgène des diamines aromatiques de formules

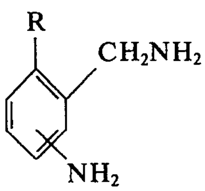

ou

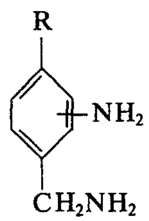

(V) (VI)

dans lesquelles R représente un groupe alkyle ayant 1 à 12 atomes de carbone, et l'on sépare thermiquement le chlorure d'acide carbamique qui est généré.

11. Utilisation de diisocyanates aromatiques de formules (I) et/ou (II) pour la préparation de matières plastiques de polyuréthanne ou d'agents de protection des plantes.

**Revendications pour l'Etat contractant: AT**

1. Procédé de préparation de diisocyanates aromatiques de formules

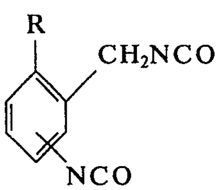

et/ou

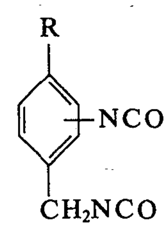

(I) (II)

caractérisé par le fait que l'on soumet à l'action du phosgène des diamines aromatiques de formules

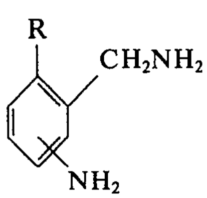

et/ou

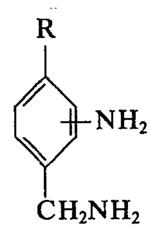

(V) (VI)

dans lesquelles R représente un groupe alkyle ayant 1 à 12 atomes de carbone, et l'on sépare thermiquement le chlorure d'acide carbamique qui est généré.

2. Procédé selon la revendication 1, caractérisé par le fait que R représente un groupe méthyle, éthyle, n-propyle, iso-propyle ou n-butyle.

3. Procédé de préparation de diisocyanates aromatiques de formules (I) et/ou (II) selon la revendication 1, caractérisé par le fait que l'on utilise comme diamines aromatiques, des composés de formules

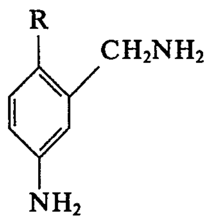

et/ou

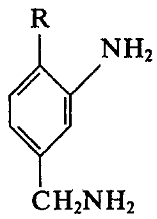

dans lesquelles R représente un groupe alkyle ayant 1 à 12 atomes de carbone.

4. Procédé de préparation de diisocyanates aromatiques de formule (I) selon la revendication 1, caractérisé par le fait que l'on utilise comme diamines aromatiques 1-méthyl-2-aminométhyl-4-amino-benzène et/ou 1-méthyl-2-aminométhyl-6-amino-benzène.

5. Procédé de préparation de diisocyanates aromatiques de formule (II) selon la revendication 1, caractérisé par le fait que l'on utilise comme diamines aromatiques 1-méthyl-4-aminométhyl-2-amino-benzène.